# EUROPEAN PATENT SPECIFICATION

(11) **EP 4 180 106 B1**
(45) Date of publication and mention of the grant of the patent: **04.11.2026**
(21) Application number: 21843513.9
(22) Date of filing: 06.07.2021
(51) Int. Cl.: B01D 9/02, C07D 277/56, C07C 59/86, C07D 223/26, B01D 9/00, C07C 51/43, C30B 7/02, C30B 7/06, C30B 29/54

(54) **REFINING METHOD**
RAFFINIERVERFAHREN
PROCÉDÉ DE RAFFINAGE

(30) Priority: 13.07.2020 WO PCT/JP2020/027266
(43) Date of publication of application: 17.05.2023
(73) Proprietor: NGK Insulators, Ltd., Nagoya city, Aichi 467-8530 (JP)
(72) Inventor: TOHNAI, Norimitsu, Suita-city, Osaka 5650871 (JP); KATO, Daiki, Nagoya-city, Aichi 467-8530 (JP); KONDO, Yoshio, Nagoya-city, Aichi 467-8530 (JP); YAMADA, Kazunari, Nagoya-city, Aichi 467-8530 (JP)
(74) Representative: Mewburn Ellis LLP
(86) International application number: PCT/JP2021/025389
(87) International publication number: WO 2022/014396

(56) References cited:
- EP-A1- 3 299 370
- EP-A1- 3 501 636
- WO-A1-2015/053297
- WO-A1-2018/034305
- CN-A- 103 333 064
- CN-A- 108 530 382
- JP-A- 2003 319 800
- JP-A- 2003 319 800
- JP-A- 2009 510 163
- JP-A- 2012 529 537
- JP-B2- 5 215 505
- US-A1- 2008 293 946
- US-A1- 2013 187 050
- US-B2- 7 122 642

## Description

### Technical Field

The present invention relates to a refining method.

### Background Art

Distillation, recrystallization, chromatography, extraction, and the like are generally known as methods for refining a target organic compound. Patent Literature 1 discloses a method for refining an organic compound using a laser beam. In Patent Literature 1, to selectively produce a metastable substance from a solution of a substance containing a stable form and a metastable form as crystal forms, metastable crystals are selectively produced by emitting a laser beam into the solution to generate bubbles and form metastable crystal nuclei.

### Citation List

### Patent Literature

PTL 1: JP 2014-189462 A

EP 3 501 636 A1 and US 7 122 642 B2 disclose infrared-induced crystallisation.

JP 5 215505 B2, CN 108 530 382 A, US 2013/187050 A1 and CN 103 333 064 A disclose crystallisation of febuxostat, cabamazepine and loxoprofen, respectively. 1

### Summary of Invention

### Technical Problem

In Patent Literature 1, however, the laser beam is emitted to generate bubbles in the solution, and no attention is paid to light of an infrared absorption wavelength.

The present invention has been made to address such an issue and mainly aims to obtain a specific crystal form from a solution of a compound dissolved in a solvent. Solution to Problem

A refining method according to the present invention is a refining method for crystallizing a compound as defined in independent claim 1.

This refining method can precipitate a specific crystal form from a solution of a compound dissolved in a solvent by adjusting the solvent for dissolving the compound, the infrared radiation emitted to the solution, and the temperature of the solution. The reason why a specific crystal form precipitates is not clear but is considered as described below. A compound with a plurality of crystal forms generally has a dissolution rate depending on the type of solvent. The dissolution rate is probably related to the ease of precipitation of crystals. Furthermore, a crystal form with higher infrared absorptivity probably has more active thermal vibrations and fewer crystal nuclei. Furthermore, the crystal form precipitated from the solution also depends on the temperature of the solution during the precipitation of crystals. It is therefore thought that the suitable conditions for precipitation of a specific crystal form depend on the solvent for dissolving the compound, the infrared radiation emitted to the solution, and the temperature of the solution.

### Brief Description of Drawings

Fig. 1 is a perspective view of a refining apparatus 1 (partially in cross section).
Fig. 2 is a partial bottom view of an infrared heater 10.
Fig. 3 is a graph of an infrared absorption spectrum of febuxostat.
Fig. 4 is a graph of an infrared absorption spectrum of loxoprofen.
Fig. 5 is a graph of an infrared absorption spectrum of carbamazepine.

### Description of Embodiments

Preferred embodiments of the invention are described in detail below.

A refining method according to the present embodiment is a refining method for crystallizing a specific crystal form of a compound with a plurality of crystal forms, wherein the compound is febuxostat, loxoprofen, or carbamazepine according to claim 1 that includes: target wavelength and a target temperature, a specific infrared wavelength and a specific temperature at which a specific crystal form precipitates from a solution of the compound dissolved in a solvent, and using an infrared radiation apparatus capable of emitting infrared radiation including the target wavelength to evaporate the solvent and precipitate the specific crystal form while irradiating the solution with infrared radiation including the target wavelength and adjusting a temperature of the solution to the target temperature.

In one example described below, a specific crystal form is precipitated by evaporating a solvent from a solution of a raw material of an organic compound X with two crystal forms a1 and a2 dissolved in the solvent. The specific crystal form is precipitated on the basis of the results of preliminary experiments. In a first preliminary experiment, it is assumed that a crystal form a1 is precipitated when a solution of the raw material of the organic compound X dissolved in a solvent p1 is irradiated with infrared radiation including a wavelength λ1 [µm] (for example, infrared radiation having a peak at the wavelength λ1) and when the temperature of the solution is adjusted to T1 [°C] to evaporate the solvent p1. In a second preliminary experiment, it is assumed that a crystal form a2 is precipitated when a solution of the raw material of the organic compound X dissolved in a solvent p1 is irradiated with infrared radiation including a wavelength λ1 [µm] and when the temperature of the solution is adjusted to T2 [°C] to evaporate the solvent p1. In such a preliminary experiment, to precipitate the crystal form a1, the solvent p1 used in the first preliminary experiment, infrared radiation including the wavelength λ1 [µm], and the temperature T1 [°C] of the solution are employed. To precipitate the crystal form a2, the solvent p1 used in the second preliminary experiment, infrared radiation including the wavelength λ1 [µm], and the temperature T2 [°C] of the solution [mg/ml] are employed. Although different crystal forms are prepared by changing the temperature of the solution with the same solvent at the same wavelength in this embodiment, the present invention is not limited to the embodiment. Different crystal forms may be prepared by appropriately changing the combination of solvent, wavelength, and temperature. For example, different crystal forms may be prepared by changing the solvent at the same wavelength and at the same temperature. Alternatively, different crystal forms may be prepared by changing the wavelength with the same solvent at the same temperature.

The wavelength λ1 [µm] is preferably determined on the basis of an infrared absorption spectrum of a crystal form and the dissolution rate of a raw material in a solvent. Crystal forms often have different infrared absorption spectra and often have different absorptivities at a given wavelength. When a solution is irradiated with infrared radiation including a certain wavelength, a crystal form with a higher absorptivity at the wavelength has more active thermal vibration than crystal forms with a lower absorptivity, has fewer crystal nuclei, and is less likely to precipitate. On the other hand, it is thought that a crystal form that can easily form crystal nuclei is different between a solvent with a high dissolution rate of a raw material and a solvent with a low dissolution rate of a raw material. Thus, the wavelength λ1 [µm] is preferably determined on the basis of an infrared absorption spectrum of a crystal form and the dissolution rate of a raw material in a solvent. For example, the infrared radiation including the wavelength λ1 [µm] may be infrared radiation having a peak at the wavelength λ1 [µm].

In the following example, a crystal form c is precipitated by evaporating a solvent q from a solution of an organic compound Y with the crystal form c dissolved in the solvent q. It is assumed that, in a preliminary experiment, the crystal form c precipitates when the solvent q is evaporated while the solution of the organic compound Y dissolved in the solvent q is irradiated with infrared radiation including a wavelength α [µm] and the temperature of the solution was adjusted to t1 [°C]. It is also assumed that the crystal form c does not precipitate and is amorphous when the solvent q is evaporated while the solution of the organic compound Y dissolved in the solvent q is irradiated with infrared radiation including a wavelength α [µm] and the temperature of the solution was adjusted to t2 [°C]. In such a case, to precipitate the crystal form c, the solvent q may be evaporated while the solution of the organic compound Y dissolved in the solvent q is irradiated with infrared radiation including a wavelength α [µm] and the temperature of the solution is adjusted to t1 [°C]. For example, the infrared radiation including the wavelength α [µm] may be infrared radiation having a peak at the wavelength α [µm].

Examples of compounds that can be refined by the refining method according to the present disclosure include febuxostat, terfenadine, indomethacin, ibuprofen, loxoprofen, caffeine, diclofenac, and carbamazepine, wherein only febuxostat, loxoprofen and carbamazepine are falling under the scope of the appended claims. The solvent for dissolving a raw material of a compound is selected from alcohol solvents selected from methanol, ethanol, 1-propanol, 2-propanol, 1-butanol, 2-butanol, isobutanol and tert-butanol, nitrile solvents selected from acetonitrile and propionitrile, ether solvents selected from diethyl ether and tetrahydrofuran, ketone solvents selected from acetone and methyl ethyl ketone, halogen solvents selected from dichloromethane and chloroform, ester solvents selected from ethyl acetate and methyl acetate, aliphatic hydrocarbon solvents selected from pentane, hexane, heptane, octane and cyclohexane, aromatic hydrocarbon solvents selected from benzene, toluene and xylene, and mixed solvents of alcohol solvents and water.

In the refining method according to the present embodiment, any infrared radiation apparatus capable of emitting infrared radiation including a wavelength λ[µm] can be used. For example, the infrared radiation apparatus may include a sheet radiator and a planar heater serving as a heat source. The infrared radiation apparatus is preferably an infrared radiation apparatus capable of emitting infrared radiation having a peak at the wavelength λ[µm], particularly infrared radiation having a peak at the wavelength λ[µm] and having a narrow half-width. Examples of such an infrared radiation apparatus include metamaterial emitters and infrared radiation apparatuses with a filter. Examples of the metamaterial emitters include emitters of a metal-insulator-metal (MIM) type, a microcavity type, a meta-atom type, and a multilayer type. Examples of the MIM type include those described in Reference 1 (JSME TED Newsletter, No. 74, pp. 7-10, 2014). The MIM type is described in detail later. Examples of the microcavity type and the meta-atom type include those described in Reference 2 (JSME TED Newsletter, No. 74, pp. 2-6, 2014). Examples of the multilayer type include those described in Reference 3 (ACS Cent. Sci., Vol. 5, pp. 319-326, 2019). Examples of the infrared radiation apparatuses with a filter include infrared heaters described in Japanese Patent No. 6442355.

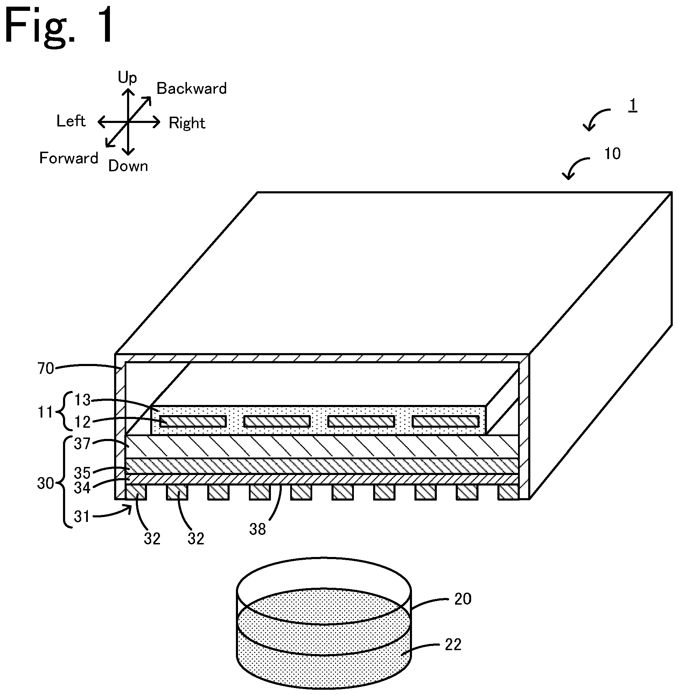

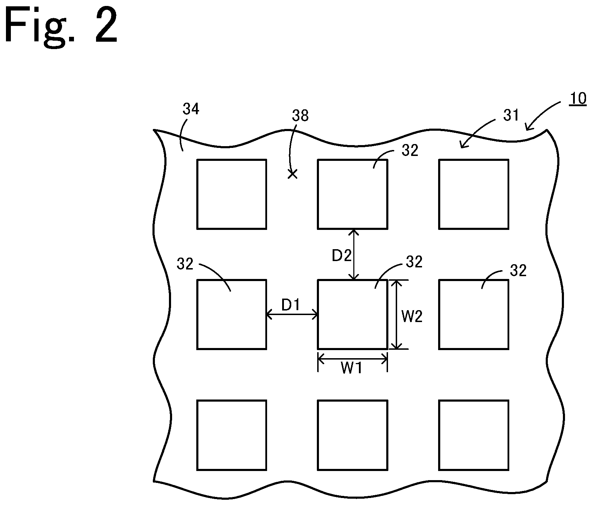

Fig. 1 is a perspective view of a refining apparatus 1 partially in cross section. Fig. 2 is a partial bottom view of an infrared heater 10. The horizontal direction, the front-back direction, and the vertical direction are as illustrated in Fig. 1.

The refining apparatus 1 is an apparatus for precipitating a specific crystal form from a solution 22 in a flat laboratory dish 20 using the infrared heater 10. The solution 22 contains a compound with a plurality of crystal forms dissolved in a solvent.

The infrared heater 10 is an example of a metamaterial emitter of the MIM type and includes a heater body 11, a structure 30, and a casing 70. The infrared heater 10 emits infrared radiation to the solution 22 in the flat laboratory dish 20 located under the infrared heater 10.

The heater body 11 is configured as a planar heater and includes a heating element 12 in which a linear member is bent in a zigzag, and a protective member 13, which is an insulator in contact with and surrounding the heating element 12. The material of the heating element 12 is, for example, W, Mo, Ta, an Fe-Cr-Al alloy, or a Ni-Cr alloy. The material of the protective member 13 is, for example, an insulating resin, such as a polyimide, or a ceramic. The heater body 11 is located inside the casing 70. Both ends of the heating element 12 are coupled to a pair of input terminals (not shown) attached to the casing 70. Electric power can be supplied to the heating element 12 from the outside through the pair of input terminals. The heater body 11 may be a planar heater with a ribbon-like heating element wound around an insulator.

The structure 30 is a sheet radiator provided under the heating element 12. The structure 30 includes a first conductor layer 31 (a metal pattern), a dielectric layer 34, a second conductor layer 35 (a metal substrate), and a supporting substrate 37 stacked in this order from the outside to the inside under the infrared heater 10. The structure 30 is located so as to close an opening in the lower portion of the casing 70.

As illustrated in Fig. 2, the first conductor layer 31 is configured as a metal pattern with a periodic structure in which metal electrodes 32 of the same shape and size are arranged at regular intervals on the dielectric layer 34. More specifically, the first conductor layer 31 is configured as a metal pattern in which a plurality of tetragonal metal electrodes 32 are arranged at regular intervals D1 in the horizontal direction and at regular intervals D2 in the front-back direction on the dielectric layer 34. The metal electrodes 32 have a shape with a thickness (a vertical height) smaller than a lateral width W1 (a width in the horizontal direction) and a longitudinal width W2 (a width in the front-back direction). The metal pattern has a transverse period Λ1 = D1 + W1 and a longitudinal period A2 = D2 + W2. It is assumed that D1 and D2 are the same, and W1 and W2 are the same. The material of the metal electrodes 32 is, for example, gold or aluminum (Al). The metal electrodes 32 are bonded to the dielectric layer 34 via an adhesive layer (not shown). The material of the adhesive layer is, for example, chromium (Cr), titanium (Ti), or ruthenium (Ru).

The dielectric layer 34 is a flat member with an upper surface bonded to the second conductor layer 35. The dielectric layer 34 is located between the first conductor layer 31 and the second conductor layer 35. A portion of the lower surface of the dielectric layer 34 on which the metal electrodes 32 are not located is a radiation surface 38 for emitting infrared radiation to an object. The material of the dielectric layer 34 is, for example, alumina (Al₂O₃) or silica (SiO₂).

The second conductor layer 35 is a metal sheet with an upper surface bonded to the supporting substrate 37 via an adhesive layer (not shown). The material of the second conductor layer 35 may be the same as the material of the first conductor layer 31. The material of the adhesive layer is, for example, chromium (Cr), titanium (Ti), or ruthenium (Ru).

The supporting substrate 37 is a flat member fixed inside the casing 70 with a fixing component or the like (not shown) and supports the first conductor layer 31, the dielectric layer 34, and the second conductor layer 35. The material of the supporting substrate 37 is, for example, a material, such as a Si wafer or glass, that can easily maintain a smooth surface, has high heat resistance, and has low thermal warping. The supporting substrate 37 may be in contact with the lower surface of the heater body 11 or may be separated from the lower surface with a space therebetween. When the supporting substrate 37 is in contact with the heater body 11, they may be bonded together.

The structure 30 functions as a metamaterial emitter with the characteristics of selectively emitting infrared radiation of a specific wavelength. The characteristics probably result from a resonance phenomenon explained by magnetic polariton. The magnetic polariton is a resonance phenomenon in which a confinement effect of a strong electromagnetic field can be produced in a dielectric (the dielectric layer 34) between two upper and lower conductors (the first conductor layer 31 and the second conductor layer 35). Thus, in the structure 30, a portion of the dielectric layer 34 between the second conductor layer 35 and the metal electrodes 32 serves as an infrared radiation source. Infrared radiation emitted from the radiation source goes around the metal electrodes 32 and is emitted to the surrounding environment from a portion of the dielectric layer 34 on which the metal electrodes 32 are not located (that is, from the radiation surface 38). In the structure 30, the materials of the first conductor layer 31, the dielectric layer 34, and the second conductor layer 35 and the shape and periodic structure of the first conductor layer 31 can be adjusted to regulate the resonance wavelength. Thus, infrared radiation emitted from the radiation surface 38 of the structure 30 characteristically has high emissivity at a specific wavelength. In the present embodiment, the material, shape, periodic structure, and the like are adjusted so that the structure 30 characteristically emits from the radiation surface 38 infrared radiation having a maximum peak with a half-width of 2.0 µm or less (preferably 1.5 µm or less, more preferably 1.0 µm or less) and with an emissivity of 0.7 or more (preferably 0.8 or more) in the wavelength range of 0.9 to 25 µm (preferably 2.5 to 25 µm (4000 to 400 cm⁻¹)). Thus, the structure 30 characteristically emits infrared radiation having a sharp maximum peak with a relatively small half-width and a relatively high emissivity. The half-width is, for example, but not limited to, preferably 2.0 µm or less, more preferably 1.5 µm or less, still more preferably 1.0 µm or less.

The casing 70 has an approximately rectangular parallelepiped shape with a space therein and with an open bottom surface. The heater body 11 and the structure 30 are located in the space inside the casing 70. The casing 70 is formed of a metal (for example, stainless steel or aluminum) to reflect infrared radiation emitted from the heating element 12.

An example of use of the refining apparatus 1 is described below. In the following example, as a specific crystal form precipitated from a solution of the organic compound X with two crystal forms a1 and a2 dissolved in a solvent, as described above, the crystal form a1 is precipitated.

First, the flat laboratory dish 20 containing the solution 22 is placed under the first conductor layer 31 of the infrared heater 10. The solution 22 contains the organic compound X dissolved in the solvent p1. Next, electric power is supplied from a power supply (not shown) through an input terminal to both ends of the heating element 12. The electric power is supplied so that the temperature of the heating element 12 reaches a preset temperature (for example, but not limited to, several hundred degrees Celsius). The heating element 12 heated to the predetermined temperature transfers energy to the surroundings by at least one of three heat transfer modes of conduction, convection, and radiation and heats the structure 30. Consequently, the structure 30 is heated to a predetermined temperature, becomes a secondary radiator, and emits infrared radiation.

In this case, a predetermined wavelength λ1 [µm] is set as a target wavelength, and infrared radiation having a peak at the wavelength λ1 [µm] is set to be emitted from the structure 30. More specifically, the intervals D1 and D2 of the metal electrodes 32 of the structure 30, the widths W1 and W2 of the metal electrodes 32, and the periods Λ1 and Λ2 of the metal pattern are set so that infrared radiation emitted from the structure 30 has a peak at a predetermined wavelength λ1 [µm]. Irradiating the solution 22 in the flat laboratory dish 20 with infrared radiation having a peak at the wavelength λ1 [µm] and adjusting the temperature of the solution to T1[°C] evaporate the solvent p1 of the solution 22 with the passage of time and finally selectively precipitate crystals of the organic compound X with the crystal form a1.

Although the infrared heater 10 is designed to mainly emit infrared radiation of a target wavelength, it is difficult to remove all radiation other than the target wavelength from the infrared radiation of the structure 30, and convective heat dissipation from components of the heater to the surroundings will occur in the atmosphere. To form an actual process, therefore, various considerations should be given to the shape of the apparatus and the like so that such associated heat flow does not excessively increase the temperature of raw materials and the like.

The refining method according to the present embodiment described in detail above can precipitate a specific crystal form from a solution of a compound dissolved in a solvent by adjusting the solvent for dissolving the compound, a peak wavelength of infrared radiation emitted to the solution, and the temperature of the solution. Furthermore, the use of the infrared heater 10 of the MIM type allows a peak wavelength of emitted infrared radiation to be designed to accurately match a target wavelength. The first conductor layer 31 of the infrared heater 10 is configured as a metal pattern with a periodic structure in which the metal electrodes 32 of the same shape and size are arranged at regular intervals. The infrared heater 10 emits infrared radiation having a peak wavelength that changes with the lateral width W1 and the longitudinal width W2 of the metal electrodes 32. The lateral width W1 and the longitudinal width W2 of the metal electrodes 32 can be accurate as designed, for example, by drawing and lift-off using a well-known electron-beam lithography system. Thus, a peak wavelength of infrared radiation emitted from the infrared heater 10 can be relatively easily and accurately adjusted to a target wavelength.

It goes without saying that the present invention should not be limited to these embodiments and can be implemented in various aspects within the technical scope of the present disclosure. The scope of the invention is defined by the appended claims.

The metal electrodes 32 are tetragonal in these embodiments but may be circular. In circular metal electrodes 32, the diameter corresponds to the lateral width W1 and the longitudinal width W2.

### EXAMPLES

### [Example 1]

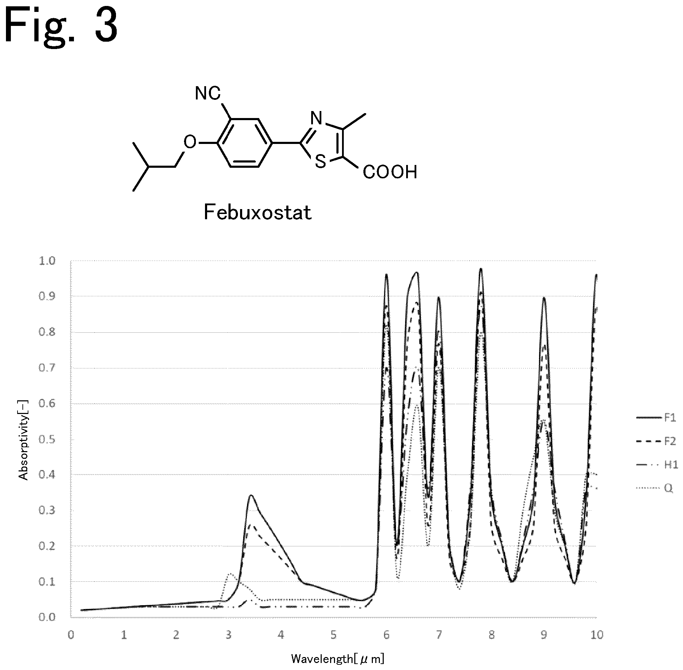

Febuxostat is known to have a plurality of crystal forms F1, F2, Q, and H1. Fig. 3 is a graph of an infrared absorption spectrum of each crystal form.

Table 1 shows the absorptivity of each crystal form at wavelengths of 3.7 and 6.7 µm in the infrared absorption spectra.

**[Table 1]**

| Compound name | Crystal form | Absorptivity (-) | | |
|---|---|---|---|---|
| | | 3.0(*µ*m) | 3.7(*µ*m) | 6.7(*µ*m) |
| Febuxostat | F1 | - | 0.27 | 0.65 |
| | F2 | - | 0.21 | 0.56 |
| | Q | - | 0.05 | 0.40 |
| | H1 | - | 0.03 | 0.53 |
| Loxoprofen | F1 | - | 0.06 | 0.40 |
| | F2 | - | 0.10 | 0.55 |
| Carbamazepine | F1 | 0.44 | - | 0.52 |
| | F2 | 0.50 | - | 0.60 |
| | F3 | 0.40 | - | 0.47 |
| | F4 | 0.47 | - | 0.55 |

A test sample was prepared by weighing 25 mg of febuxostat (product code F0847, Tokyo Chemical Industry Co., Ltd.) into a flat laboratory dish (ϕ32 mm x 16 mm), adding 1 mL of isopropanol (IPA), heating the febuxostat on a hot plate at 80°C for 2 minutes, and dissolving the febuxostat with slight stirring. The temperature of the solution was adjusted to 40°C while the test sample was irradiated with infrared radiation including a wavelength of 6.7 µm (infrared radiation having a peak at a wavelength of 6.7 µm) (radiation source temperature: 400°C), and this state was maintained to evaporate the solvent and precipitate crystals. Infrared radiation was emitted from the infrared heater 10 of the MIM type. In the infrared heater 10, the first conductor layer 31 (a layer having circular metal electrodes 32) made of Au had a height h of 50 nm. The dielectric layer 34 made of Al₂O₃ had a thickness d of 190 nm. The second conductor layer 35 made of Au had a height f of 100 nm. The circular metal electrodes 32 had a diameter (corresponding to W1 and W2) of 2.16 µm. The intervals between the metal electrodes (corresponding to D1 and D2) were 1.84 µm. The period (corresponding to Λ1 and Λ2) was 4.0 µm. Infrared radiation having a peak at a wavelength of 6.7 µm (half-width: 0.5 µm) was emitted. The crystal form of the precipitated crystals was identified as F2 by XRD analysis. The XRD analysis was performed with an X-ray diffractometer (product name: Ultima IV, Rigaku).

### [Example 2]

Crystals were precipitated in the same manner as in Example 1 except that the temperature of the solution to precipitate crystals was changed from 40°C to 55°C. The crystal form of the precipitated crystals was identified as H1 by XRD analysis.

### [Example 3]

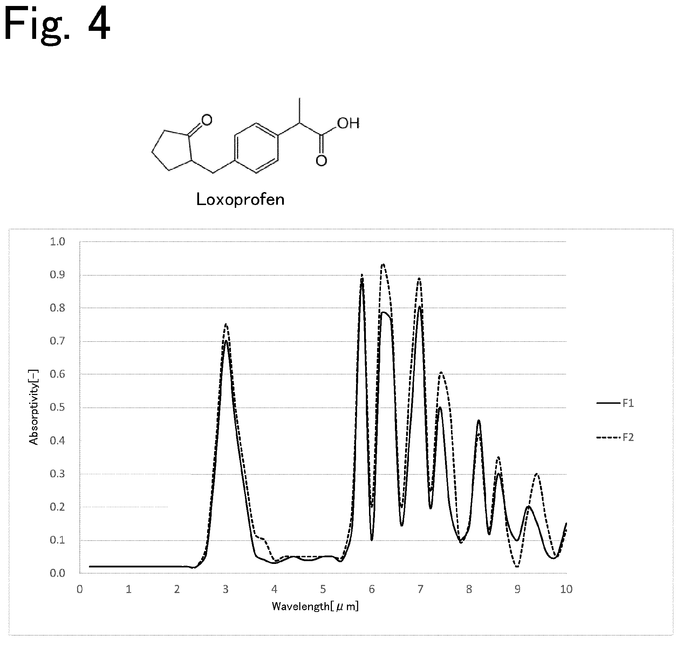

Loxoprofen is known to have a plurality of crystal forms F1 and F2. Fig. 4 is a graph of an infrared absorption spectrum of each crystal form. Table 1 shows the absorptivity of each crystal form at wavelengths of 3.7 and 6.7 µm in the infrared absorption spectra.

A test sample was prepared by weighing 5 mg of loxoprofen (product code L0244, Tokyo Chemical Industry Co., Ltd.) into a flat laboratory dish (ϕ32 mm x 16 mm), adding 1 mL of isopropanol (IPA), heating the loxoprofen on a hot plate at 80°C for 1 minute, and dissolving the loxoprofen with slight stirring. The temperature of the solution was adjusted to 80°C while the test sample was irradiated with infrared radiation including a wavelength of 6.7 µm, and this state was maintained to evaporate the solvent and precipitate crystals. The temperature of the solution was adjusted by placing the test sample on a heating plate with a Peltier element and using the temperature control function of the heating plate. The crystal form of the precipitated crystals was identified as F1 by XRD analysis.

### [Example 4]

Crystals were precipitated in the same manner as in Example 3 except that the temperature of the solution to precipitate crystals was changed from 80°C to 55°C. A heating plate was not used. The crystal form of the precipitated crystals was identified as F2 by XRD analysis.

### [Example 5]

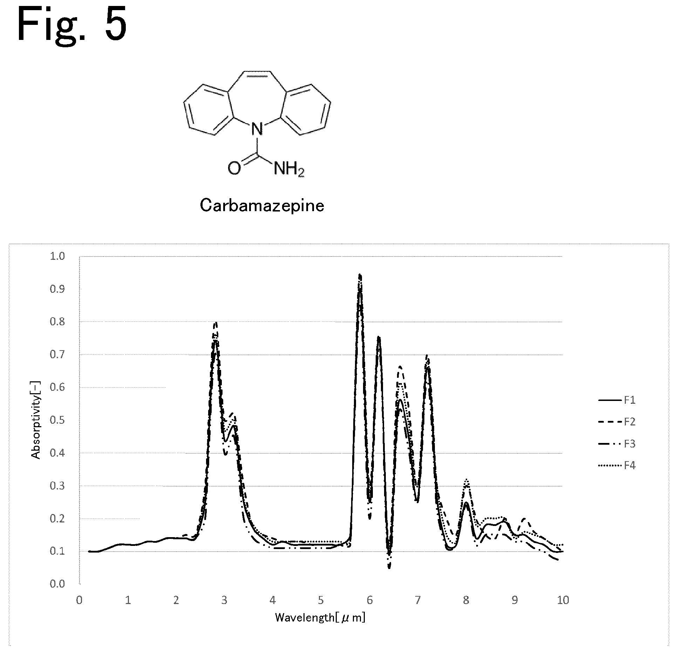

Carbamazepine is known to have a plurality of crystal forms F1, F2, F3, and F4. Fig. 5 is a graph of an infrared absorption spectrum of each crystal form. Table 1 shows the absorptivity of each crystal form at wavelengths of 3.0 and 6.7 µm in the infrared absorption spectra.

A test sample was prepared by weighing 25 mg of carbamazepine (product code C1095, Tokyo Chemical Industry Co., Ltd.) into a flat laboratory dish (ϕ32 mm x 16 mm), adding 1 mL of isopropanol (IPA), heating the carbamazepine on a hot plate at 80°C for 1 minute, and dissolving the carbamazepine with slight stirring. The temperature of the solution was adjusted to 55°C while the test sample was irradiated with infrared radiation including a wavelength of 3.0 µm, and this state was maintained to evaporate the solvent and precipitate crystals. A heating plate was not used. The crystal form of the precipitated crystals was identified as F2 by XRD analysis.

In Example 5, the first conductor layer 31 of the infrared heater 10 had a height h of 49 nm. The dielectric layer 34 had a thickness d of 44 nm. The second conductor layer 35 had a height f of 200 nm. The circular metal electrodes 32 had a diameter (corresponding to W1 and W2) of 0.54 µm. The intervals between the metal electrodes (corresponding to D1 and D2) were 0.46 µm. The period (corresponding to Λ1 and A2) was 1.0 µm. Infrared radiation having a peak at a wavelength of 3.0 µm (half-width: 0.5 µm) was emitted.

### [Example 6]

Crystals were precipitated in the same manner as in Example 5 except that the temperature of the solution to precipitate crystals was changed from 55°C to 80°C. The temperature of the solution was adjusted to 80°C on a heating plate with a Peltier element. The crystal form of the precipitated crystals was identified as F3 by XRD analysis.

The results of Examples 1 to 6 are summarized in Table 2.

**[Table 2]**

| Example | Compound name | Wavelength (*µ*m) | Solvent | Concentration (mg/mL) | Solution temperature (°C) | Crystal form |
|---|---|---|---|---|---|---|
| 1 | Febuxostat | 6.7 | IPA | 25 | 40 | F2 |
| 2 | | | | | 55 | H1 |
| 3 | Loxoprofen | 6.7 | IPA | 5 | 80 | F1 |
| 4 | | | | | 55 | F2 |
| 5 | Carbamazepine | 3.0 | IPA | 25 | 55 | F2 |
| 6 | | | | | 80 | F3 |

Examples 1 to 6 show that the solvent, wavelength, and temperature could be appropriately combined to precipitate a compound with a different crystal form in the solution of the compound. From another perspective, even with the same solvent and wavelength, the temperature could be changed to precipitate a compound with a different crystal form.

Examples 1 and 2 show that, to precipitate febuxostat of the crystal form F1, as described in Example 1, IPA may be used as a solvent, infrared radiation with a peak wavelength of 6.7 µm may be emitted, and the temperature may be adjusted to 40°C. To precipitate febuxostat of the crystal form F2, as described in Example 2, IPA may be used as a solvent, infrared radiation with a peak wavelength of 6.7 µm may be emitted, and the temperature may be adjusted to 55°C.

Examples 3 and 4 show that, to precipitate loxoprofen of the crystal form F1, as described in Example 3, IPA may be used as a solvent, infrared radiation with a peak wavelength of 6.7 µm may be emitted, and the temperature may be adjusted to 80°C. To precipitate loxoprofen of the crystal form F2, as described in Example 4, IPA may be used as a solvent, infrared radiation with a peak wavelength of 6.7 µm may be emitted, and the temperature may be adjusted to 55°C.

Examples 5 and 6 show that, to precipitate carbamazepine of the crystal form F2, as described in Example 5, IPA may be used as a solvent, infrared radiation with a peak wavelength of 3.0 µm may be emitted, and the temperature may be adjusted to 55°C. To precipitate carbamazepine of the crystal form F3, as described in Example 6, IPA may be used as a solvent, infrared radiation with a peak wavelength of 3.0 µm may be emitted, and the temperature may be adjusted to 80°C.

### Industrial Applicability

The present invention is used to selectively obtain a specific crystal form of a compound with a plurality of crystal forms.

### Reference Signs List

1 refining apparatus, 10 infrared heater, 11 heater body, 12 heating element, 13 protective member, 20 flat laboratory dish, 22 solution, 30 structure, 31 first conductor layer, 32 metal electrode, 34 dielectric layer, 35 second conductor layer, 37 supporting substrate, 38 radiation surface, 70 casing, W1 lateral width, W2 longitudinal width, D1, D2 interval

## Claims

1. A refining method for crystallizing a specific crystal form of a compound with a plurality of crystal forms, wherein the compound is febuxostat, loxoprofen, or carbamazepine, the method being **characterized in that** the method comprises:
setting, as a target wavelength and a target temperature, a specific infrared wavelength and a specific temperature at which a specific crystal form precipitates from a solution of the compound dissolved in a solvent that is selected from alcohol solvents selected from methanol, ethanol, 1-propanol, 2-propanol, 1-butanol, 2-butanol, isobutanol and tert-butanol, nitrile solvents selected from acetonitrile and propionitrile, ether solvents selected from diethyl ether and tetrahydrofuran, ketone solvents selected from acetone and methyl ethyl ketone, halogen solvents selected from dichloromethane and chloroform, ester solvents selected from ethyl acetate and methyl acetate, aliphatic hydrocarbon solvents selected from pentane, hexane, heptane, octane and cyclohexane, aromatic hydrocarbon solvents selected from benzene, toluene and xylene, and mixed solvents of the alcohol solvents and water; and
using an infrared radiation apparatus capable of emitting infrared radiation including the target wavelength to evaporate the solvent and precipitate the specific crystal form while irradiating the solution with infrared radiation including the target wavelength and adjusting a temperature of the solution to the target temperature.

2. The refining method according to Claim 1, wherein the infrared radiation apparatus includes a sheet radiator and a planar heater serving as a heat source.

3. The refining method according to any one of Claims 1 to 2, wherein the infrared radiation apparatus can emit infrared radiation having a peak at the target wavelength.

4. The refining method according to Claim 3, wherein the infrared radiation apparatus emits infrared radiation having a peak at the target wavelength from a structure composed of a metal pattern, a dielectric layer, and a metal substrate stacked in this order from the outside to the inside, the metal pattern includes metal electrodes of the same shape and size arranged at regular intervals on the dielectric layer, and a peak wavelength of the infrared radiation changes depending on a width of the metal electrodes.

## Patentansprüche

1. Raffinierverfahren zum Kristallisieren einer speziellen Kristallform einer Verbindung mit einer Vielzahl von Kristallformen, wobei die Verbindung Febuxostat, Loxoprofen oder Carbamazepin ist, wobei das Verfahren **dadurch gekennzeichnet ist, dass** das Verfahren Folgendes umfasst:
das Einstellen einer speziellen Infrarot-Wellenlänge und einer speziellen Temperatur als Ziel-Wellenlänge und Ziel-Temperatur, bei der eine spezielle Kristallform aus einer Lösung der Verbindung ausfällt, die in einem Lösungsmittel gelöst ist, das aus Alkohol-Lösungsmitteln, ausgewählt aus Methanol, Ethanol, 1-Propanol, 2-Propanol, 1-Butanol, 2 Butanol, Isobutanol und tert-Butanol; Nitril-Lösungsmitteln, ausgewählt aus Acetonitril und Propionitril; Ether-Lösungsmitteln, ausgewählt aus Diethylether und Tetrahydrofuran; Keton-Lösungsmitteln, ausgewählt aus Aceton und Methylethylketon; Halogen-Lösungsmitteln, ausgewählt aus Dichlormethan und Chloroform; Ester-Lösungsmitteln, ausgewählt aus Ethylacetat und Methylacetat; aliphatischen Kohlenwasserstoff-Lösungsmitteln, ausgewählt aus Pentan, Hexan, Heptan, Octan und Cyclohexan; aromatischen Kohlenwasserstoff-Lösungsmitteln, ausgewählt aus Benzol, Toluol und Xylol; und Lösungsmittelgemischen der Alkohol-Lösungsmittel und Wasser, ausgewählt ist; und
das Verwenden einer Infrarot-Strahlungsvorrichtung, die dazu in der Lage ist, Infrarot-Strahlung, einschließlich der Ziel-Wellenlänge, abzustrahlen, um das Lösungsmittel zu verdampfen und die spezielle Kristallform auszufällen, während die Lösung mit Infrarot-Strahlung, einschließlich der Ziel-Wellenlänge, bestrahlt wird, das und Einstellen der Temperatur der Lösung auf die Ziel-Temperatur.

2. Raffinierverfahren nach Anspruch 1, wobei die Infrarot-Strahlungsvorrichtung eine Platten-Strahlungsvorrichtung und eine plattenförmige Heizvorrichtung, die als Wärmequelle dient, umfasst.

3. Raffinierverfahren nach einem der Ansprüche 1 bis 2, wobei die Infrarot-Strahlungsvorrichtung Infrarot-Strahlung mit einem Peak bei der Ziel-Wellenlänge ausstrahlen kann.

4. Raffinierverfahren nach Anspruch 3, wobei die Infrarot-Strahlungsvorrichtung Infrarot-Strahlung, die einen Peak bei der Ziel-Wellenlänge aufweist, aus einer Struktur ausstrahlt, die aus einer Metallstruktur, einer dielektrischen Schicht und einem Metallsubstrat besteht, die in dieser Reihenfolge von außen nach innen gestapelt sind, wobei die Metallstruktur Metallelektroden derselben Form und Größe umfasst, die in regelmäßigen Abständen auf der dielektrischen Schicht angeordnet sind, und wobei sich die Peak-Wellenlänge der Infrarot-Strahlung in Abhängigkeit von einer Breite der Metallelektroden ändert.

## Revendications

1. Procédé de raffinage pour cristalliser une forme cristalline spécifique d'un composé ayant une pluralité de formes cristallines, dans lequel le composé est fébuxostat, loxoprofène ou carbamazépine, le procédé étant **caractérisé en ce que** le procédé comprend :
l'établissement, en tant que longueur d'onde cible et température cible, d'une longueur d'onde infrarouge spécifique et d'une température spécifique auxquelles une forme cristalline spécifique est précipitée à partir d'une solution du composé dissous dans un solvant qui est sélectionné parmi des solvants alcooliques sélectionnés parmi méthanol, éthanol, 1-propanol, 2-propanol, 1-butanol, 2-butanol, isobutanol et tert-butanol, des solvants nitriles sélectionnés parmi acétonitrile et propionitrile, des solvants éthers sélectionnés parmi éther diéthylique et tétrahydrofurane, des solvants cétones sélectionnés parmi acétone et méthyléthylcétone, des solvants halogénés sélectionnés parmi dichlorométhane et chloroforme, des solvants esters sélectionnés parmi acétate d'éthyle et acétate de méthyle, des solvants hydrocarbures aliphatiques sélectionnés parmi pentane, hexane, heptane, octane et cyclohexane, des solvants hydrocarbures aromatiques sélectionnés parmi benzène, toluène et xylène, et des solvants mixtes constitués des solvants alcooliques et d'eau ; et
l'utilisation d'un appareil de rayonnement infrarouge capable d'émettre un rayonnement infrarouge comportant la longueur d'onde cible pour évaporer le solvant et précipiter la forme cristalline spécifique, tout en irradiant la solution avec un rayonnement infrarouge comportant la longueur d'onde cible et tout en ajustant une température de la solution à la température cible.

2. Procédé de raffinage selon la revendication 1, dans lequel l'appareil de rayonnement infrarouge comporte un radiateur en feuille et un chauffage plan servant de source de chaleur.

3. Procédé de raffinage selon l'une quelconque des revendications 1 à 2, dans lequel l'appareil de rayonnement infrarouge peut émettre un rayonnement infrarouge présentant un pic à la longueur d'onde cible.

4. Procédé de raffinage selon la revendication 3, dans lequel l'appareil de rayonnement infrarouge émet un rayonnement infrarouge présentant un pic à la longueur d'onde cible à partir d'une structure composée d'un motif métallique, d'une couche diélectrique et d'un substrat métallique empilés dans cet ordre de l'extérieur vers l'intérieur, le motif métallique comporte des électrodes métalliques de même forme et de même taille disposées à intervalles réguliers sur la couche diélectrique, et une longueur d'onde pic du rayonnement infrarouge varie en fonction d'une largeur des électrodes métalliques.
